# EUROPEAN PATENT SPECIFICATION

(11) **EP 2 419 937 B1**
(45) Date of publication and mention of the grant of the patent: **17.09.2014**
(21) Application number: 10714784.5
(22) Date of filing: 14.04.2010
(51) Int. Cl.: A61F 2/44

(54) **MINIMALLY INVASIVE EXPANDABLE CONTAINED VERTEBRAL IMPLANT**
MINIMALINVASIVES ERWEITERBAR ENTHALTENES VERTEBRAL-IMPLANTAT
IMPLANT VERTÉBRAL CONFINÉ POUVANT ÊTRE EXPANSÉ, À INVASION MINIMALE

(30) Priority: 16.04.2009 US 424880
(43) Date of publication of application: 22.02.2012
(73) Proprietor: Warsaw Orthopedic, Inc., Warsaw, IN 46581 (US)
(72) Inventor: CAPOTE, Marco, D., Lafayette Colorado 80026 (US); DEWEY, Jonathan, M., Sunnyvale California 94085 (US); DROCHNER, Thomas, E., Memphis Tennessee 38103 (US); DVORAK, Marcel, Vancouver British Columbia V65 IN3 (CA); FISHER, Charles, G., Vancouver British Columbia VGN Y2 (CA); MELKENT, Anthony, J., Memphis Tennessee 38111 (US); MILLER, Keith, E., Germantown Tennessee 38138 (US); RAMPERSAUD, Y. Raja, Toronto Ontario M6S 1G6 (CA); STEWART, Ronald, G., Germantown Tennessee 38139 (US)
(74) Representative: Viering, Jentschura & Partner Patent- und Rechtsanwälte
(86) International application number: PCT/US2010/031071
(87) International publication number: WO 2010/120911

(56) References cited:
- WO-A1-2006/053210
- WO-A1-2008/148210
- FR-A1- 2 774 581
- US-A- 3 867 728
- US-A1- 2003 191 536
- US-A1- 2004 249 462

## Description

### BACKGROUND

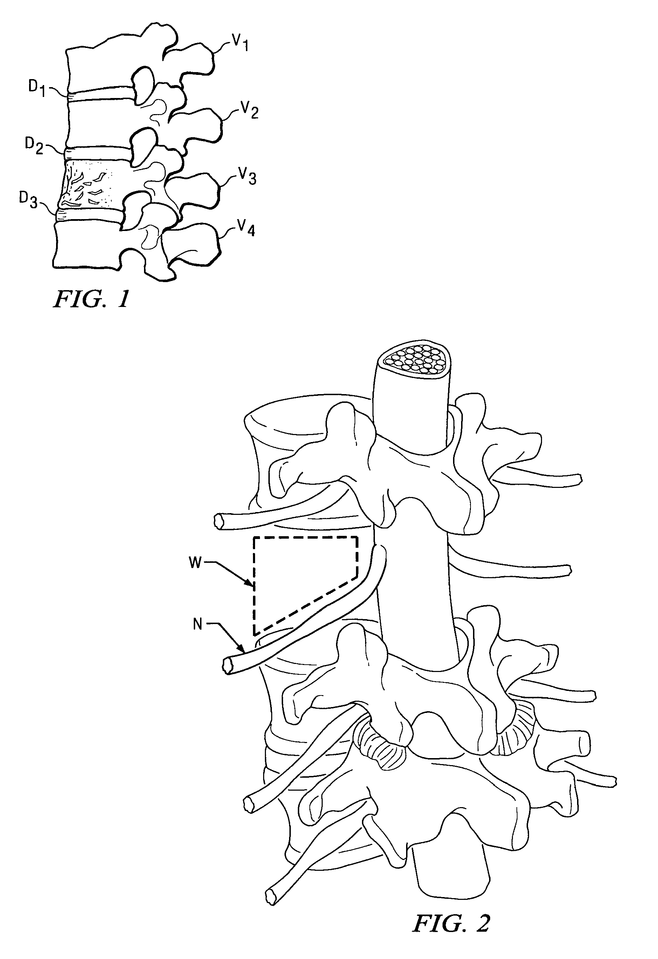

It is sometimes necessary to remove one or more vertebrae, or a portion of the vertebrae, from the human spine in response to various pathologies. For example, one or more of the vertebrae may become damaged as a result of tumor growth, or may become damaged by a traumatic or other event. Removal, or excision, of a vertebra may be referred to as a vertebrectomy. Excision of a generally anterior portion, or vertebral body, of the vertebra may be referred to as a corpectomy. An implant is usually placed between the remaining vertebrae to provide structural support for the spine as a part of a corpectomy or vertebrectomy. FIG. 1 illustrates four vertebrae, V₁-V₄ of a typical lumbar spine and three spinal discs, D₁-D₃. As illustrated, V₃ is a damaged vertebra and all or a part of V₃ could be removed to help stabilize the spine. If removed along with spinal discs D₂ and D₃, an implant may be placed between vertebrae V₂ and V₄. In some cases, the implant inserted between the vertebrae is designed to facilitate fusion between remaining vertebrae. In other cases, especially when treating tumors, the ultimate goal of the procedure is spinal stability, regardless of fusion. A successful procedure may decrease pain, preserve or enhance neurological function and allow a patient greater mobility without an external orthosis. Sometimes an implant is designed to replace the function of the excised vertebra and discs. All or part of more than one vertebra may be damaged and require removal and replacement in some circumstances. If only a portion of a vertebral body and adjacent discs are removed and replaced, the procedure is called a hemi-vertebrectomy.

Many implants are known in the art for use in vertebrectomy and corpectomy procedures. One class of implants is sized to directly replace the vertebra or vertebrae that are being replaced, without in situ expansion. Another class of implants is inserted in a collapsed state and then expanded once properly positioned. Expandable implants may be advantageous because they allow for a smaller incision and entry path when positioning an implant. A smaller incision may be particularly useful with a posterior approach, as illustrated in FIG 2. FIG. 2 is an illustration from the posterior of a portion of a human spine, with one thoracic vertebra removed. To support the remaining vertebral structure, an implant may be placed through the window W, avoiding the nerve root N. The nerve root N, may be mobilized to increase the size of the window W slightly, but excess movement may risk damage to the nerve root N. Therefore, for a posterior approach, an initially small expandable implant may have particular utility. A posterior approach may be preferred for patients with circumferenlial tumors or for patients more susceptible to the risks associated with a more extensive anterior approach. Similarly, initially small implants enabling minimal tissue disruption may be useful from any surgical approach to reduce trauma to surrounding tissues and to enhance patient recovery.

Once in position and expanded, it may be advantageous for a corpectomy or vertebrectomy implant to, as nearly as possible, fill the space vertically between the remaining vertebrae and laterally among the remaining soft tissues. Lateral expansion may increase the contact area between the implant and vertebral endplates. This expansion may reduce the potential for subsidence of the device into the adjacent vertebrae. However, it may be important that the lateral expansion not impinge on the spinal cord or nerve roots. In some instances, it may be useful to control lateral expansion to a particular distance or volume.

Expandable implants may also be useful in replacing long bones or portions of appendages such as the legs and arms, or a rib or other bone that is generally, though not necessarily, longer than it is wide. Examples include, but are not limited to a femur, tibia, fibula, humerus, radius, ulna, phalanges, clavicle, and any of the ribs. Use of the mechanisms described and claimed herein are equally applicable to treatment or repair of such bones or appendages. Similarly, expandable implants may be useful in at least some spinal fusion procedures where a spinal disc is replaced without replacing a vertebral body. US 2004/249462 A1 discloses an artificial intervertebral disc, WO 2006/053210 A1 discloses a vertebral lift device.

### SUMMARY

The invention provides an expandable medical implant according to claim 1. Further embodiments are described in the dependent claims.

### BRIEF DESCRIPTION OF THE DRAWINGS

FIG. 1 is an elevation view of a segment of a lumbar spine.
FIG. 2 is a posterior perspective view of a portion of a human spine.
FIG. 3 is a perspective view of an embodiment of an expandable medical implant.
FIG. 4 is a perspective view of the laterally rigid component embodiment of FIG. 3.
FIG. 5 is a plan view of the superior end of the implant of FIG. 3 with a portion of the membrane removed to illustrate an end of the laterally expandable component.
FIG. 6 is an elevation view of an embodiment of an expandable medical implant.
FIG. 7 is an elevation view of an embodiment of an expandable medical implant.
FIG. 8 is an elevation view of an embodiment of an expandable medical implant.
FIG. 9 is an elevation view of an embodiment of an expandable medical implant.
FIG. 10 is a perspective view of an embodiment of an expandable medical implant in an unexpanded state.
FIG. 11 is a perspective view of the laterally rigid component embodiment of FIG. 10 in an unexpanded state.
FIG. 12 is a perspective view of an embodiment of the expandable medical implant of FIG. 10 in an expanded state.
FIG. 13 is a perspective view of the laterally rigid component embodiment of FIG. 10 in an expanded state.
FIG. 14 is a cross-sectional view of an embodiment of an expandable medical implant.
FIG. 15 is a cross-sectional view of an embodiment of an expandable medical implant.
FIG. 16 is a cross-sectional view of an embodiment of an expandable medical implant.
FIG. 17 is a perspective view illustrating an embodiment of an expandable medical implant being introduced through a generally posterior approach.
FIG. 18 is a perspective view illustrating an embodiment of an expandable medical implant expanded between vertebrae.

### DETAILED DESCRIPTION

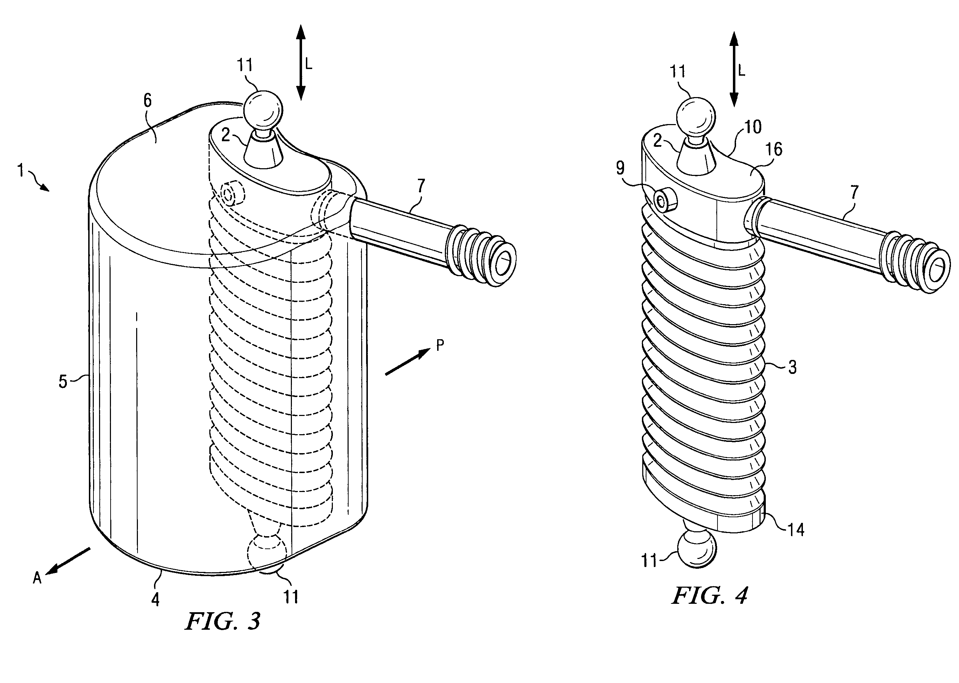

FIGS. 3-5 illustrate one embodiment of an expandable medical implant 1 for supporting skeletal structures. The expandable medical implant 1 shown is depicted in an expanded state. The illustrated expandable medical implant 1 includes a membrane 5 defining a volume and having an upper surface 6, an opposite lower surface 4, and a first side 10 between the upper and lower surfaces 6, 4. A laterally rigid component 3 is shown coupled at least at one point to the first side 10 of the membrane 5. In some embodiments, an adhesive is applied between the membrane and the laterally rigid component to couple the parts to one another. Some embodiments may not include a coupling between the laterally rigid component 3 and the first side 10. The illustrated laterally rigid component 3 of FIGS. 3-5 is a bellows. The laterally rigid component 3 is substantially contained within the volume of the membrane 5. Coupling of the membrane 5 to the laterally rigid component at the first side 10 may restrict lateral expansion of the membrane 5 in at least a first direction. In some embodiments, the expandable medical implant 1 will be oriented with the membrane 5 toward the anterior of a spinal column, as noted by the letter A, and with the laterally rigid component 3 toward the posterior of the spinal column, as noted by the letter P. The specified orientations are for illustrative purposes only and may be altered in various other embodiments. The longitudinal axis, or linear expansion direction L, of the laterally rigid component 3 is also illustrated.

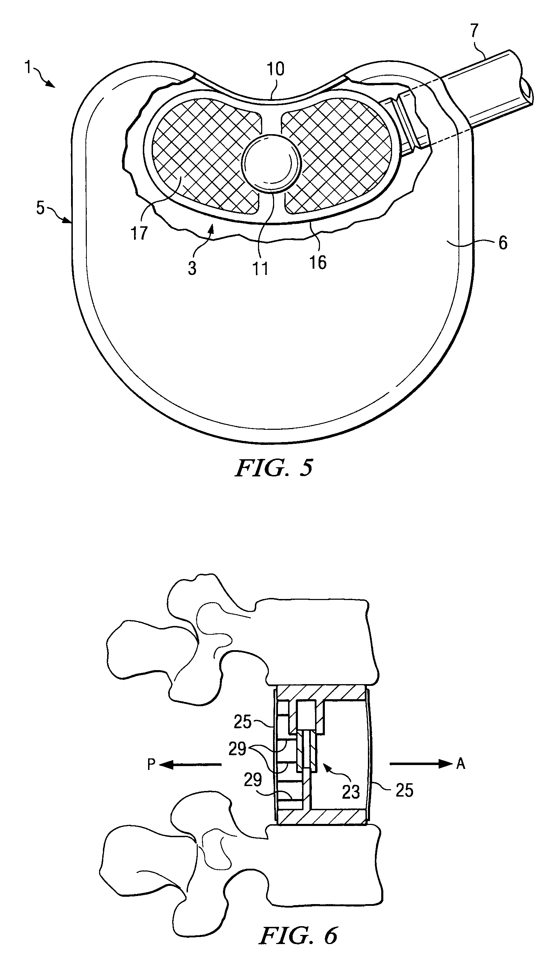

The membrane 5 is illustrated in an expanded state in FIGS. 3 and 5. The membrane 5 of some embodiments is configured to be placed between vertebrae and expanded such that the upper surface 6 contacts a first vertebra and the opposite lower surface 4 contacts a second vertebra to provide support between the vertebrae. Lateral expansion of the membrane 5 is also accomplished in some embodiments. For example, in FIGS. 3 and 5, anterior expansion, as well as medial-lateral expansion, and intervening radial expansions, are illustrated. As used herein, the term lateral means directions approximately normal to the linear expansion direction L.

The membrane 5 may be constructed, in whole or in part, of a non-permeable material. The membrane 5 may include compliant or non-compliant balloon materials such as those commonly used to manufacture coronary and Kyphoplasty medical devices. Such materials may include, but are not limited to, mylar, rubber, polyurethane, vinyl, latex, polyethylenes, ionomer, and polytetrapthalate (PET), as well as less flexible materials such as Kevlar®, PEBAX®, stainless steel, titanium, nickel-titanium alloys, and other metals and alloys and/or ceramics. A compliant membrane may include reinforcing to limit one or both of the size and shape of the membrane to a clinically advantageous extent. A non-compliant membrane may expand more elastically to more completely fill an irregular opening, depending on the amount of material introduced into the membrane.

Likewise the membrane 5 may be constructed, in whole or in part, of a permeable material, which allows a certain amount of a fill material 100 to pass through the membrane 5. All or a portion may be made permeable by fabricating a material, including but not limited to, the membrane materials listed above, into a fabric, weave, mesh, composite, bonded fiber assembly, or any other manufacture known to those skilled in the art. For example, all or part of the upper surface 6 and the opposite lower surface 4 may be constructed of a permeable material to allow fill material 100 to move through the membrane 5 and to come into contact with vertebrae.

FIG. 4 illustrates an embodiment of the laterally rigid component 3 in an expanded state. As shown in FIGS. 3-5, the cross-sectional shape of the embodiment of the laterally rigid component 3 is concave-convex. However, in other embodiments, the cross-sectional shape of the laterally rigid component 3 may be any functional shape, such as but not limited to, generally round, oval, rectangular, triangular, polygonal, or combinations of these shapes.

The laterally rigid component 3 illustrated in FIG. 4, which is a bellows, is defined herein as "laterally rigid" because it appreciably resists forces applied lateral to its linear axis L. Other devices that expand linearly and resist forces applied laterally to their linear axes L, are devices of FIGS. 6-16. These, and any other devices that provide resistance to lateral forces, are contemplated in embodiments of the present invention and are within the scope of the claims herein. Resistance to lateral forces may be useful in the illustrated embodiments for protecting posterior neural structures such as, but not limited to, the spinal cord, spinal canal, and nerve roots.

FIGS. 3 and 4 illustrate a nozzle 2 extending from the laterally rigid component 3. A similar nozzle, alternatively or in addition, may extend from the opposite end of the laterally rigid component 3. The illustrated nozzle 2 is open to the interior of the laterally rigid component 3. A balloon 11 is shown extending from an open, distal end of the nozzle 2. The balloon 11 shown is in fluid communication with the interior of the laterally rigid component 3. The nozzle 2 and balloon 11 of some embodiments are configured to extend from the laterally rigid component 3 and into an endplate of an adjacent vertebra. The balloon 11 may be filled with a material, such as a flowable material, to assist in attachment of the lateral rigid component 3 to the adjacent vertebra. The balloon 11 may additional have a therapeutic effect on the vertebra. For example, and without limitation, the balloon 11, alone or in combination with the nozzle 2, may help to stabilize the vertebra. The material used to inflate the balloon 11 may be a curable material or may be a material that is used to expand the balloon 11, but does not cure in place. Once expanded, the balloon 11 may also receive additional materials that permanently fill the balloon 11, or that have an additional therapeutic effect on the vertebra. Any of the materials for use in the balloon 11 may also be a fill material 100 as described in detail below. In addition to the nozzle 2 or balloon 11, an end of an embodiment of the laterally rigid component 3 my include teeth, spikes, ridges, indentations, roughening, knurling, or any other device for enhancing fixation between a vertebra and the laterally rigid component 3. Any of the balloon 11 embodiments noted herein may be non-permeable or permeable to some degree and may be elastic or constrained to a particular size or shape.

FIGS. 4 and 5 illustrate an upper cap 16 of the laterally rigid component 3. FIG. 4 additionally shows a lower cap 14. The upper and lower caps 16, 14 may be non-permeable or permeable to some degree, with similar functions to, and made from similar materials to, the membrane 5 discussed above. The upper and lower caps 16, 14 may be made from the same material as the rest of the laterally rigid component 3, or may be made from a separate material. FIG. 5 illustrates an upper cap 16 made from an at least partially permeable woven or mesh material. As illustrated, a mesh end 17 is integrated into the upper cap 16 to allow passage of some of the fluid or fill material 100 to contact an adjacent vertebra. Alternatively, or in addition, the upper cap 16 may be expanded or stretched into contact with an adjacent vertebra. The upper and lower caps 16, 14 of some embodiments directly contact adjacent vertebra, or alternatively may contact inner portions of the membrane 5.

In some embodiments, elasticity in the material of the laterally rigid component 3 may sever as a biasing force to bias the laterally rigid component 3 toward an expanded or unexpanded state, as may be advantageous in various circumstances. For example, it may be advantageous to bias the laterally rigid component 3 toward an unexpanded state to provide a low profile device for insertion. Other devices may be used to expand the device as needed. In other circumstances, it may be preferred to bias the laterally rigid component 3 toward an expanded state. With such an embodiment, another component may be used to keep the laterally rigid component 3 in an unexpanded state while it is inserted. Then following insertion, the laterally rigid component 3 may be released and allowed to increase toward its expanded state.

The laterally rigid component 3 may be coupled to the membrane at more than one point along the first side 10 of the membrane 5. For example and without limitation, FIG. 6 illustrates an embodiment of the invention with multiple tethers 29 between a membrane 25 and a laterally rigid component 23. The illustrated embodiment is depicted in cross-section and shows the tethers 29 limiting expansion of the membrane 25 to the posterior P. In other embodiments, the restriction of expansion may be for another desired direction or for a combination of directions. The cross-section also illustrates a telescoping mechanism that is part of the laterally rigid component 23. Each tether 29 may be made of any biocompatible material. The tethers 29 illustrated are strands, but in other embodiments may be loops, hooks, staples, fasteners of any kind, or any other component capable of connecting the membrane 25 with the laterally rigid component 23. The telescoping mechanism may include a fluid communication opening to drive or hold expansion, or may merely be a passive component that provides a linear travel trajectory and lateral rigidity to the implant.

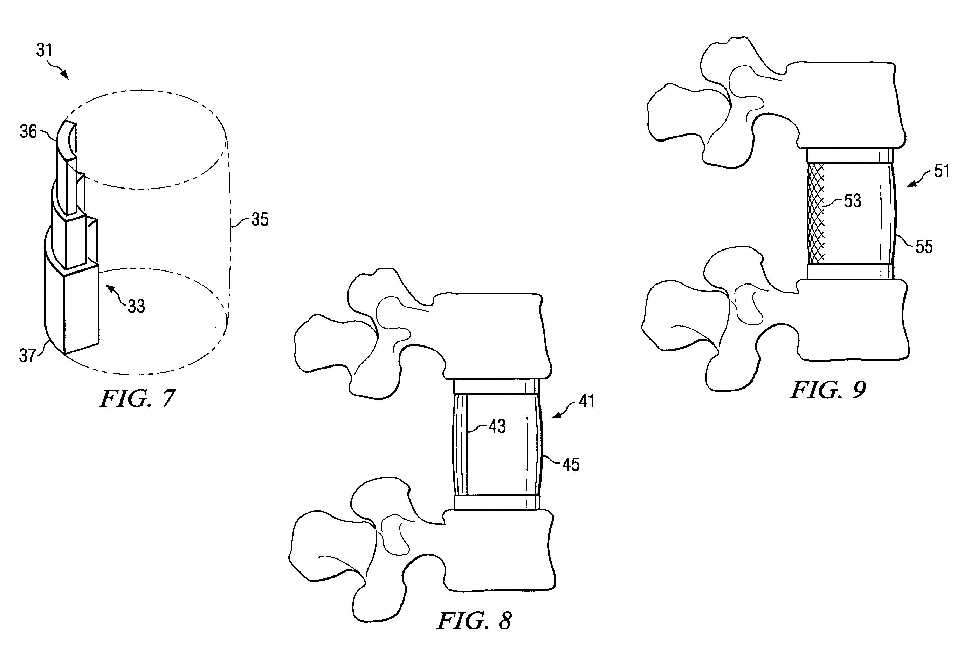

FIG. 7 shows a laterally rigid component 33 coupled with a membrane 35. The laterally rigid component 33 is a telescoping shield. The term telescoping may include devices that have interlocking members that slide relative to one another regardless of whether members fit within the periphery of other members. The laterally rigid component 33 is a passively expanding component that does not drive or hold vertical expansion of the expandable medical implant 31. The laterally rigid component 33 may be coupled to the membrane 35 at a first point 36 near an upper surface of the membrane 35 and at a second point 37 near a lower surface of the membrane 35.

FIGS. 8 and 9 show embodiments of expandable medical implants 41, 51 between vertebrae. The illustrated embodiments include a laterally rigid component 43, 53 imbedded into, formed with, juxtaposition with, or otherwise integral with at least a first side of the respective membranes 45, 55. These embodiments may provide for continuous coupling between laterally rigid components 43, 53 and respective membranes 45, 55. The laterally rigid component 43 may be an elastic band that resists lateral expansion of the membrane 45. The laterally rigid component 43 may further be compressed with the membrane 45 prior to expansion for reduced profile insertion into a vertebral space. The laterally rigid component 53 may include wires, stiff filaments, or the like that resist lateral expansion of the membrane 55. For example and without limitation, the laterally rigid component may include a fabric, weave, mesh, composite, bonded fiber assembly, or any other manufacture known to those skilled in the art, of wires, stiff filaments, or the like.

Returning to FIGS. 3-5, for example, expansion of the laterally rigid component 3 may be realized by introduction of a fluid into the laterally rigid component 3, may result from elasticity in the material from which the component is made, from internal or external biasing devices, or from any other effective device for generating expansion. Embodiments of the expandable medical implant 1 include a port 7 for receiving a fluid. As used in association with this function, a fluid may be a paste, gel, liquid, suspension, granular mixture, or similar substance. A substance as described herein will be considered a fluid even if it later cures or hardens to a non-fluidic state. As illustrated in FIGS. 3-5, the port 7 is connected to the laterally rigid component 3. In other embodiments, the port 7 may connect to the membrane 5, or to another portion of the expandable medical implant 1. The fluid received through the port 7 in some circumstances will drive expansion of the expandable medical implant 1. This may be accomplished by pressurizing the laterally rigid component 3 or the membrane 5, or by pressurizing both. In other embodiments, the fluid may be used to maintain expansion generated by some other force such as expansion from elasticity in the material from which the laterally rigid component 3 or membrane 5 are made, from biasing devices internal or external to the laterally rigid component 3 or membrane 5, or from any other effective device for generating expansion. The port 7 may also serve as an extension of the expandable medical implant 1 useful in manipulating the expandable medical implant 1 into a clinically effective location. The port 7 may be cut, broken, or otherwise removed from the expandable medical implant 1 once implanted.

As shown in FIG. 4, a transfer opening 9 is provided between the laterally rigid component 3 and the membrane 5. In some embodiments, the transfer opening 9 is a hole through which a fluid or fill material 100, or both, may pass. Passage may occur in either direction between the laterally rigid component 3 and the membrane 5 in various embodiments. As illustrated, fluid, fill material 100 or both enter the laterally rigid component 3 through the port 7 and pass through the transfer opening 9 into the membrane 5. In some embodiments, a valve is provided at the transfer opening 9 to control flow between the laterally rigid component 3 and the membrane 5. The valve may be controlled by direct manipulation or through instrumentation connected through the port 7. The valve, a combination of a series of valves, or configurations of conduits may be used to simultaneously or selectively pass fill material 100 through or into one or all of the laterally rigid component 3, the balloons 11, and the membrane 5. Valves used with any embodiment may be pressure activated valves that pass material without further user intervention once a threshold pressure is reached.

The fill material 100 may enter the expandable medical implant 1 as a fluid, and then harden or cure in the implant. In some embodiments, a non-hardenable and non-curing fluid is used to expand, or to hold expansion in, the implant or one or some of the components of the implant. A fill material 100 may then be introduced into at least the membrane 5 to provide support between the upper surface 6 and the lower surface 4. The fill material 100 may be a paste, gel, liquid, suspension, granular mixture, or similar substance. Non-limiting examples of fill materials 100 include bone cement, paste, morselized allograft, autograft, or xenograft bone, ceramics, or various polymers. An example bone cement is polymethylmethacrylate (PMMA), which may be made from methylmethacrylate, polymethylmethacrylate, esters of methacrylic acid, or copolymers containing polymethylmethacrylate and polystyrene. Additional non-limiting examples of the fill material 100 include semi-rigid flowable or hardenable material such as silicone or various types of urethane materials. It should further be understood that other types of fill materials 100 which are not necessarily hardenable or curable may be used in association with the present invention. For example, the fill material may comprise beads or small particles or grains of material, some of which may, in aggregate, achieve a harder consistency as a result of interlocking or compaction. In some embodiments, the fill material may also include a bone growth promoting substance. The use and components of such bone growth promoting substances are described in more detail below.

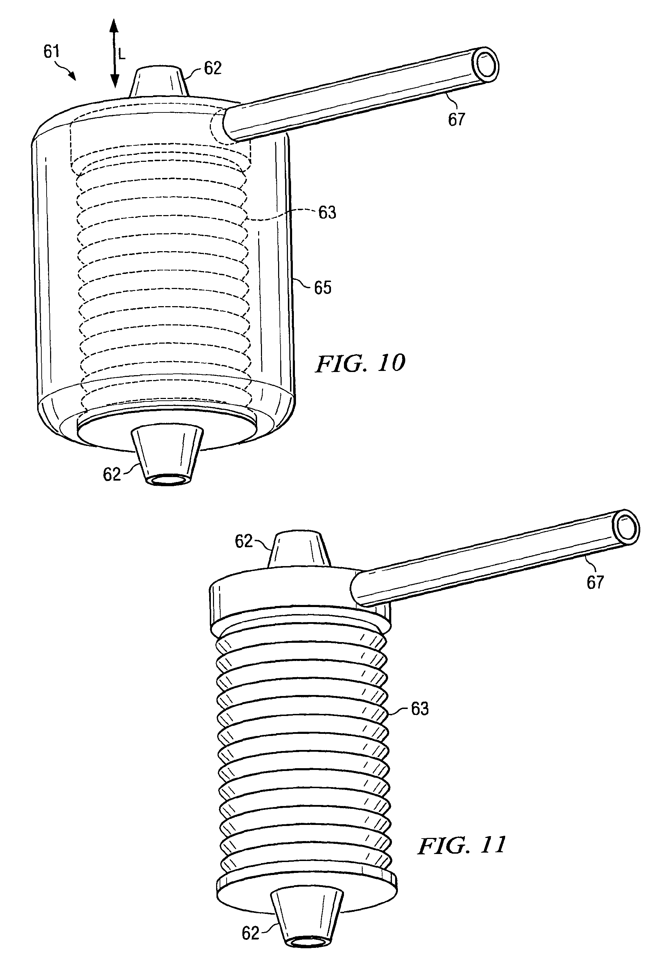

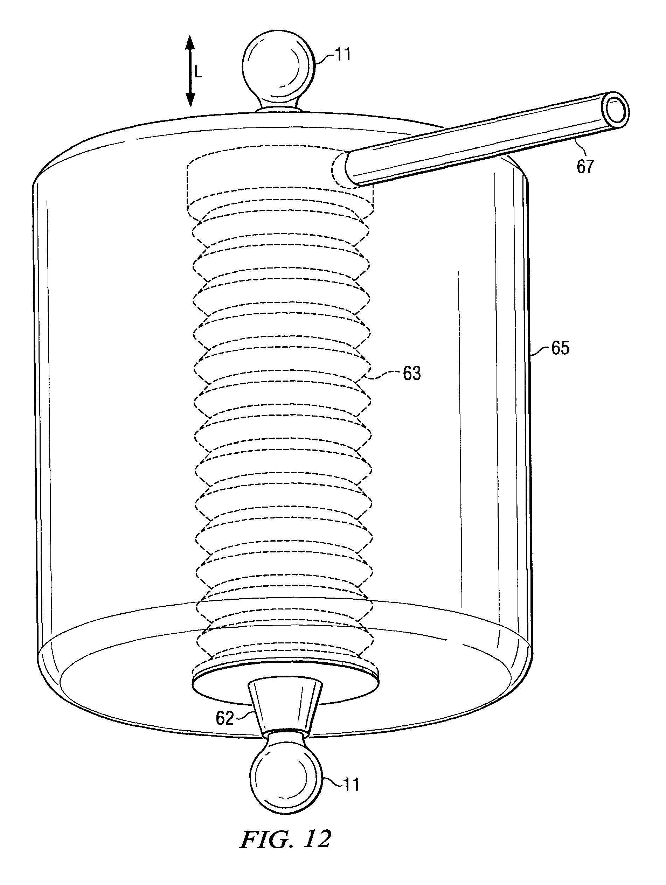

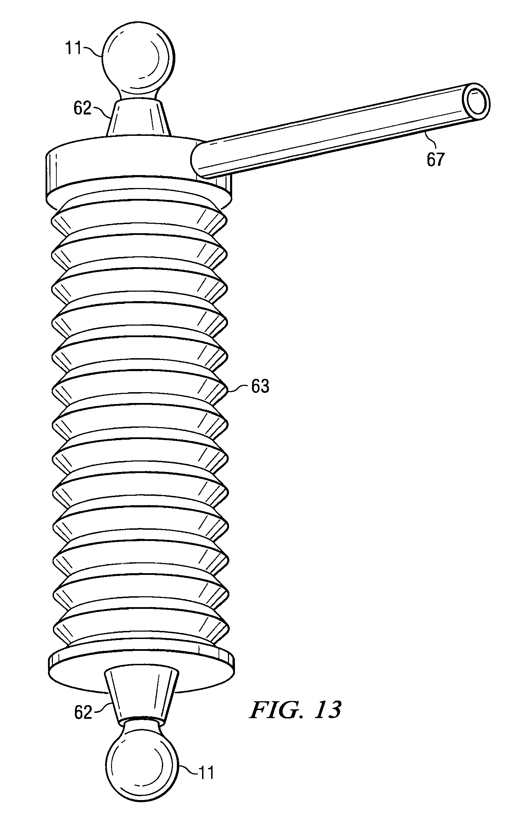

FIGS. 10-13 illustrate an embodiment of an expandable medical implant 61. In FIGS. 10 and 11, the expandable medical implant 61 is in a contracted or unexpanded state, and in FIGS. 12 and 13, an expanded state. FIGS. 10 and 12 show a membrane 65 defining a volume and ends of a linearly expandable component 63 coupled to the membrane and substantially contained within the volume of the membrane 65. The linearly expandable component 63 may also be laterally rigid, as defined herein, in some embodiments. Similarly, properties and functions of the linearly expandable component 63 described herein may be applied to any of the other laterally rigid or linearly expandable components illustrated through other embodiments. The linearly expandable component 63 illustrated in FIGS. 10-13 is configured to receive a fluid that drives linear expansion of the expandable medical implant 61 or maintains linear expansion of the expandable medical implant 61. In the illustrated embodiment, a fluid may be introduced through a port 67. As noted with previous embodiments, the fluid may be merely for expansion and retention, or may be a component of the fill material 100. As used in association with this function, a fluid may be a paste, gel, liquid, suspension, granular mixture, or similar substance. A substance as described herein will be considered a fluid even if it later cures or hardens to a non-fluidic state. The port 67 may be used to handle the expandable medical implant 61 or to guide the implant into a position where it can be effectively deployed. A nozzle 62 extends from each end of the linearly expandable component 63. The illustrated nozzles 62 are open to the interior of the linearly expandable component 63.

As is graphically illustrated in the state change between FIGS. 10 and 12, the membrane 65 is also configured to expand laterally. Lateral expansion of the membrane 65 may be accomplished by introducing fill material 100 through the linearly expandable component 63 and into the membrane 65 through an opening or valve in the linearly expandable component 63. Alternatively or in addition, fill material 100 may be introduced directly into the membrane 65 through a port formed in the membrane 65. Lateral expansion is directed or restricted by coupling the membrane 65 at one or more points to the linearly expandable component 63. Coupling may be accomplished by use of an adhesive or tethers, or by any effective device, including those specified in association with the coupling of the membranes of FIGS. 3-9. Additionally, lateral expansion may be directed or restricted by use of an expandable medical implant with another implant or instrument. For example, and without limitation, an instrument may be placed between the expandable medical implant and a spinal cord or other structure during expansion of the expandable medical implant. After expansion, and in some instances after expansion and hardening of a fill material, the instrument is removed from the patient.

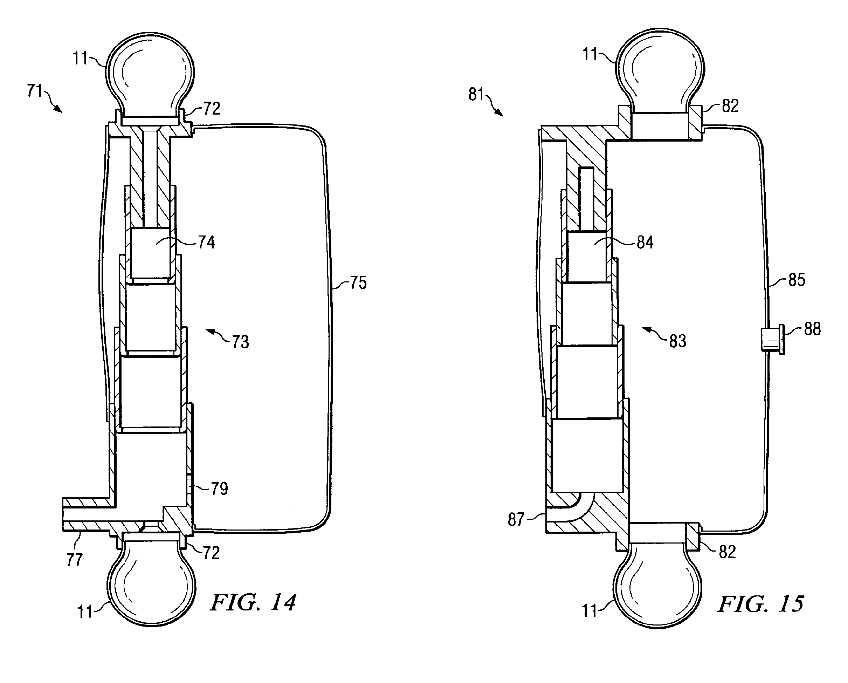

FIG. 14 illustrates an expandable medical implant 71 with particular fluid and fill material directing features. The cross-sectional view of FIG. 14 shows a linearly expandable and laterally rigid component 73 within a volume defined by a membrane 75. A port 77 for receiving a fill material 100 such as cement is in fluid communication with the laterally rigid component 73. A chamber 74 is defined within the laterally rigid component 73. In the embodiment shown, the chamber 74 is in fluid communication with each of the nozzles 72, and respective balloons 11 that extend from the nozzles 72. Therefore, when the laterally rigid component 73 is pressurized with a fluid or fill material 100, the laterally rigid component 73 is forced open or held in an open state, and the balloons 11 are pressurized to expand into adjacent vertebrae. The laterally rigid component 73 also includes a transfer opening 79 through which fluid or fill material 100 may be passed to fill the membrane 75. In some embodiments, the transfer opening 79 may include a valve to control flow between the laterally rigid component 73 and the membrane 75. The valve may be controlled by direct manipulation or through instrumentation connected through the port 77.

FIG. 15 shows an expandable medical implant 81 with particular fluid and fill material directing features. The cross-sectional view of FIG. 15 shows a linearly expandable and laterally rigid component 83 within a volume defined by a membrane 85. A first port 87 for receiving a force to drive expansion is in fluid communication with the laterally rigid component 83. The force to drive expansion in this or any of the other disclosed embodiments may be a fluid pressure, a push from a structural member, a rotation of a member internal to the laterally rigid component 83, or any force effective to expand the component or hold expansion of the component. A chamber 84 is defined within the laterally rigid component 83 to contain and direct the force. The membrane 85 includes a second port 88 through which fluid or fill material 100 may be passed to fill the membrane 85. In the embodiment shown, the membrane 85 is in fluid communication with each of the nozzles 82, and respective balloons 11 that extend from the nozzles 82. Therefore, when the membrane 85 is pressurized with a fluid or fill material 100, the membrane 85 is moved to an expanded state, and the balloons 11 are pressurized to expand into adjacent vertebrae.

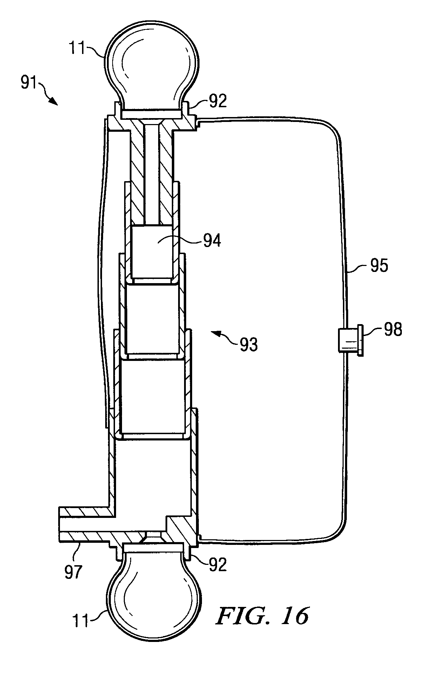

FIG. 16 depicts an expandable medical implant 91 with particular fluid and fill material directing features. The cross-sectional view of FIG. 16 shows a linearly expandable and laterally rigid component 93 within a volume defined by a membrane 95. A first port 97 for receiving a force to drive expansion is in fluid communication with the laterally rigid component 93. The force to drive expansion in this or any of the other disclosed embodiments may be a fluid pressure, a push from a structural member, a rotation of a member internal to the laterally rigid component 93, or any force effective to expand the component or hold expansion of the component. A chamber 94 is defined within the laterally rigid component 93 to contain and direct the force. In the embodiment shown, the chamber 94 is in fluid communication with each of the nozzles 92 and respective balloons 11 that extend from the nozzles 92. Therefore, when the laterally rigid component 93 is pressurized with a fluid or fill material 100, the laterally rigid component 93 is forced open or held in an open state, and the balloons 11 are pressurized to expand into adjacent vertebrae. In some embodiments the balloons 11 may be filled separately from the laterally rigid component 93. The membrane 95 includes a second port 98 through which fluid or fill material 100 may be passed to fill the membrane 95.

Each of the embodiments disclosed herein may be described as a means for occupying a vertebral space. The means for occupying a vertebral space may include a containment means defining a volume, and a linearly expandable means substantially contained within the volume of the containment means. In some embodiments, the containment means is a membrane that is placed into the vertebral space in an unexpanded state and is then expanded either or both linearly and laterally to occupy a desired portion of the vertebral space. The linearly expandable means of some embodiments is configured to receive a fluid or fill material that drives linear expansion or maintains linear expansion of the means for occupying a vertebral space. The linearly expandable means may also linearly expand the containment means in some embodiments. The containment means of some embodiments is configured to occupy a vertebral space to the extent of soft tissues that surround the spinal column. These soft tissues may include, but are not limited to, one or more of ligaments, muscles, vessels, arteries, and neural structures.

For embodiments of each of the implants disclosed herein, the size or shape of the membrane may be limited to only fill a particular portion of a vertebral space. For example, and without limitation, an implant may be configured to only occupy a lateral portion of a vertebral space to accomplish a hemi-vertebrectomy. Implants may be alternatively shaped to occupy other, limited portions of a vertebral space.

Embodiments of the implant in whole or in part may be constructed of biocompatible materials of various types. Examples of implant materials include, but are not limited to, non-reinforced polymers, carbon-reinforced polymer composites, PEEK and PEEK composites, low density polyethylene, shape-memory alloys, titanium, titanium alloys, cobalt chrome alloys, stainless steel, ceramics and combinations thereof. If a trial instrument or implant is made from radiolucent material, radiographic markers can be located on the trial instrument or implant to provide the ability to monitor and determine radiographically or fluoroscopically the location of the body in the spinal space. In some embodiments, the implant or individual components of the implant may be constructed of solid sections of bone or other tissues. Tissue materials include, but are not limited to, synthetic or natural autograft, allograft or xenograft, and may be resorbable or non-resorbable in nature. Examples of other tissue materials include, but are not limited to, hard tissues, connective tissues, demineralized bone matrix and combinations thereof.

FIG. 1 illustrates four vertebrae, V₁-V₄, of a typical lumbar spine and three spinal discs, D₁-D₃, and FIG. 2 depicts a portion of a typical thoracic spine. Embodiments of the invention may be applied to the lumbar spinal region, and embodiments may also be applied to the cervical or thoracic spine or between other skeletal structures.

Some embodiments may also include supplemental fixation devices in addition to or as part of the expandable medical implant for further stabilizing the anatomy. For example, and without limitation, rod and screw fixation systems, anterior, posterior, or lateral plating systems, facet stabilization systems, spinal process stabilization systems, and any devices that supplement stabilization may be used as a part of or in combination with the expandable medical implant. Embodiments of the invention may be useful in at least some spinal fusion procedures where a spinal disc is replaced without replacing a vertebral body.

A method embodiment of the invention includes making an expandable medical implant by providing a linearly and laterally expandable membrane and coupling a laterally rigid component that is linearly expandable to the inside of the membrane at least at one side of the membrane. A coupling to the inside of the membrane may include imbedding a laterally rigid component into the wall of a membrane or forming the devices together or juxtaposition with each other. Embodiments of the method also include forming a port in the expandable medical implant for receiving a fluid to drive or hold linear expansion of the expandable medical implant, and forming a port in the expandable medical implant for receiving a fill material that laterally expands the membrane. For example, and without limitation, the expandable medical implant 1 of FIGS. 3-5 provides a linearly and laterally expandable membrane 5 with a laterally rigid component 3 coupled inside of the membrane 5. A port 7 provides for receiving fluid to drive or hold linear expansion of the expandable medical implant 1. The drive fluid may be a separate drive fluid, as specified for the embodiment of FIG. 15, or it may be a fill material 100 in a fluidic state prior to hardening. The transfer opening 9 of FIG. 4 is an example of a port in the expandable material that laterally expands the membrane 5. Other exemplary ports for receiving material to laterally expand the membrane 5 include second ports 88, 98 of FIGS. 15 and 16. The device of FIGS. 3-5 illustrates a device made by forming a port 7 for receiving a fluid to drive or hold linear expansion in the laterally rigid component 3 and forming a port (transfer opening 9) for receiving fill material out of the laterally rigid component 3 and into the membrane 5.

In some methods, the acts of forming the ports noted are accomplished by forming a single port in the membrane. For example, and without limitation, in the embodiments of FIGS. 7-9, a single port into the membrane 35, 45, 55 would provide both a port for receiving a fluid to drive or hold linear expansion and a port for receiving a fill material 100. As the membranes 35, 45, 55 are filled with a fluid fill material, the laterally rigid components 33, 43, 53 are also linearly expanded. Further introduction of fill material also laterally expands the membranes 35, 45, 55.

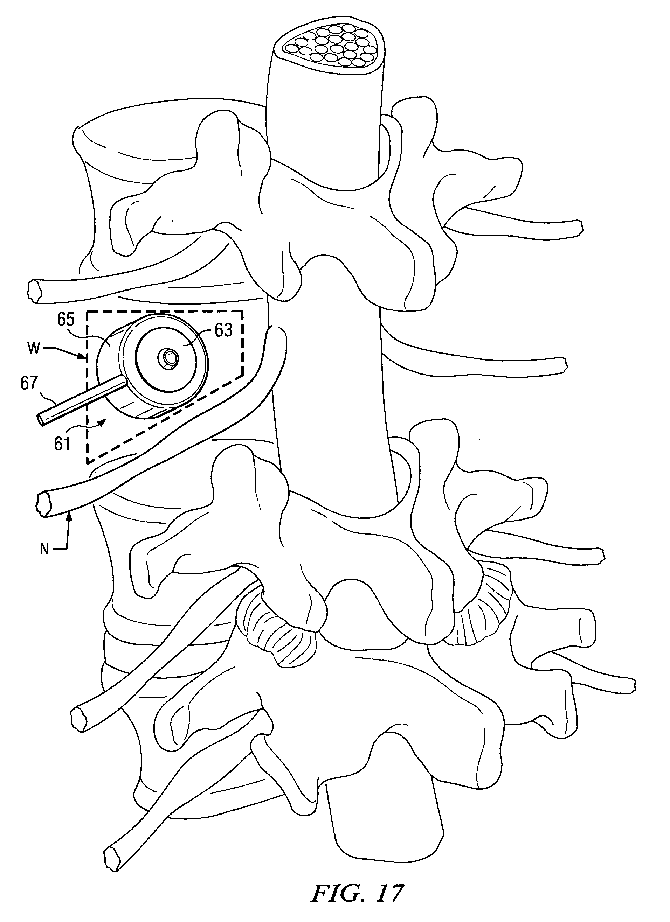

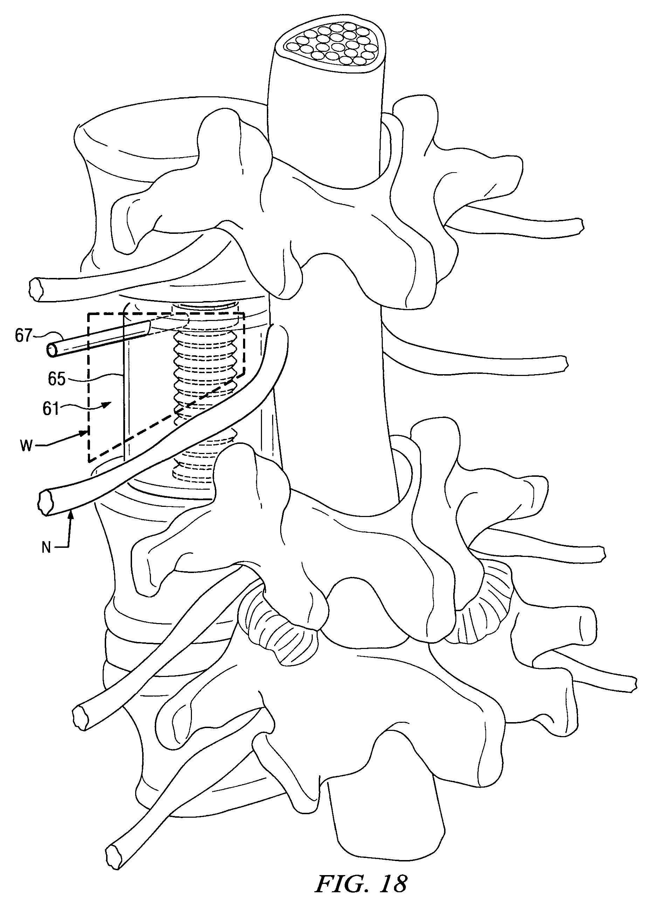

FIGS. 17 and 18 illustrate the introduction and expansion of an embodiment of an expandable medical implant 61 into a vertebral space. The implant is placed through the window W, as described above, and past a nerve root N. The window W is created among the soft tissues of a segment of the spinal column. The expandable medical implant introduced is a vertebral prosthesis that includes the laterally rigid expandable component 63 and the adjacent membrane 65 with an upper surface and an opposite lower surface. FIG. 17 shows a distal end of the expandable medical implant being introduced through the window W first. In other embodiments, another portion of the expandable medical implant may be introduced first and other translational and rotational manipulations may be conducted on the implant as it is introduced.

Once the expandable medical implant 61 has been introduced through the window W and positioned appropriately in the vertebral space, it may be expanded linearly and laterally. A linearly and laterally expanded implant is illustrated in FIG. 18. The embodiment illustrated may be expanded by introducing a fluid through the port 67 and into the laterally rigid component 63. Introducing fluid through the port 67 may be caused by pressure or force generated by a syringe, injector, multi-stage injector, central pressurization reservoir, or any effective system or device. The fluid introduced to expand the laterally rigid component 63 may be fluid that is introduced only to expand the component, or in some instances, is a final fill material 100. When the fluid introduce to expand the implant is not, or is not a component of, the fill material 100, the fluid may be removed from the implant after the fill material 100 has been introduced. The introduced fluid may be one part of a multi-part fill material. The fluid or a fill material 100 may further be passed from the laterally rigid component 63 into the membrane 65 to linearly and laterally expand the membrane 65. In some embodiments, flow into the laterally rigid component 63 and the membrane 65 are separately controlled so that one or the other may be selectively filled independent from the other. In some embodiments, an additional port, or a port replacing the port 67 in the laterally rigid component 63, may connect with the membrane 65 directly.

In some embodiments, introduction of the fill material 100, by whatever mechanism that is effective, results in a non-fluidic structural support between the upper and lower surfaces of the membrane 65. Even though a fill material 100 may be initially introduced as a fluid, embodiments of the invention provide for the fill material 100 to cure or harden as noted above and provide structural support. Other fill materials 100 may have a non-fluidic state as a result of interlocking of the particles with one another or with the membrane 65, or may be non-fluidic as a response to compaction.

The expandable medical implant is shown in FIGS. 17 and 18 as being implanted from a generally posterior approach. However, embodiments of the invention may include implantation from any surgical approach, including but not limited to, posterior, lateral, anterior, transpedicular, lateral extracavitary, in conjunction with a laminectomy, in conjunction with a costotransversectomy, or by any combination of these and other approaches.

Various method embodiments of the invention are described herein with reference to particular expandable medical implants. However, in some circumstances, each disclosed method embodiment may be applicable to each of the expandable medical implants, or to some other implant operable as disclosed with regard to the various method embodiments.

Terms such as lower, upper, anterior, posterior, inferior, superior, lateral, medial, contralateral, and the like have been used herein to note relative positions. However, such terms are not limited to specific coordinate orientations, but are used to describe relative positions referencing particular embodiments. Such terms are not generally limiting to the scope of the claims made herein.

While embodiments of the invention have been illustrated and described in detail in the disclosure, the disclosure is to be considered as illustrative and not restrictive in character. All changes and modifications that come within the spirit of the invention are to be considered within the scope of the disclosure.

## Claims

1. An expandable medical implant (1, 31, 41, 51, 61, 71, 81, 91) for supporting skeletal structures comprising:
a laterally and longitudinally expandable membrane (5, 25, 35, 45, 55, 65, 75, 85, 95) defining a volume and having an upper surface, an opposite lower surface, and a first side between the upper and lower surfaces;
and a laterally rigid and longitudinally expandable component (3, 23, 33, 43, 53, 63, 73, 83, 93) coupled at least at one point to the first side, the laterally rigid and longitudinally expandable component (3, 23, 33, 43, 53, 63, 73, 83, 93) substantially contained within the volume of the laterally and longitudinally expandable membrane (5, 25, 35, 45, 55, 65, 75, 85, 95);
wherein the laterally and longitudinally expandable membrane (5, 25, 35, 45, 55, 65, 75, 85, 95) is coupled to the laterally rigid and longitudinally expandable component (3, 23, 33, 43, 53, 63, 73, 83, 93) at its first side to restrict lateral expansion of the laterally and longitudinally expandable membrane (5, 25, 35, 45, 55, 65, 75, 85, 95) in at least a first direction.

2. The expandable medical implant (1, 31, 41, 51, 61, 71, 81, 91) of claim 1 wherein the laterally and longitudinally expandable membrane (5, 25, 35, 45, 55, 65, 75, 85, 95) is, at least in part, a permeable laterally and longitudinally expandable membrane (5, 25, 35, 45, 55, 65, 75, 85, 95).

3. The expandable medical implant (1, 31, 41, 51, 61, 71, 81, 91) of claim 1 wherein the laterally rigid and longitudinally expandable component (3, 23, 33, 43, 53, 63, 73, 83, 93) is coupled to the laterally and longitudinally expandable membrane (5, 25, 35, 45, 55, 65, 75, 85, 95) at more than one point along the first side of the laterally and longitudinally expandable membrane (5, 25, 35, 45, 55, 65, 75, 85, 95).

4. The expandable medical implant (1, 31, 41, 51, 61, 71, 81, 91) of claim 1 wherein the laterally rigid and longitudinally expandable component (3, 23, 33, 43, 53, 63, 73, 83, 93) is coupled to the laterally and longitudinally expandable membrane (5, 25, 35, 45, 55, 65, 75, 85, 95) at a first point near the upper surface of the laterally and longitudinally expandable membrane (5, 25, 35, 45, 55, 65, 75, 85, 95) and at a second point near the lower surface of the laterally and longitudinally expandable membrane (5, 25, 35, 45, 55, 65, 75, 85, 95).

5. The expandable medical implant (1, 31, 41, 51, 61, 71, 81, 91) of claim 1 wherein the laterally rigid and longitudinally expandable component (3, 23, 33, 43, 53, 63, 73, 83, 93) is coupled to the laterally and longitudinally expandable membrane (5, 25, 35, 45, 55, 65, 75, 85, 95) continuously along the first side of the laterally and longitudinally expandable membrane (5, 25, 35, 45, 55, 65, 75, 85, 95).

6. The expandable medical implant (1, 31, 41, 51, 61, 71, 81, 91) of claim 1 wherein the laterally rigid and longitudinally expandable component (3, 23, 33, 43, 53, 63, 73, 83, 93) is imbedded in at least a first side of the laterally and longitudinally expandable membrane (5, 25, 35, 45, 55, 65, 75, 85, 95).

7. The expandable medical implant (1, 31, 41, 51, 61, 71, 81, 91) of claim 1, further comprising an adhesive between the laterally and longitudinally expandable membrane (5, 25, 35, 45, 55, 65, 75, 85, 95) and the laterally rigid and longitudinally expandable component (3, 23, 33, 43, 53, 63, 73, 83, 93).

8. The expandable medical implant (1, 31, 41, 51, 61, 71, 81, 91) of claim 1 further comprising at least one tether between the laterally and longitudinally expandable membrane (5, 25, 35, 45, 55, 65, 75, 85, 95) and the laterally rigid and longitudinally expandable component (3, 23, 33, 43, 53, 63, 73, 83, 93).

9. The expandable medical implant (1, 31, 41, 51, 61, 71, 81, 91) of claim 1 wherein the laterally rigid and longitudinally expandable component (3, 23, 33, 43, 53, 63, 73, 83, 93) is a linearly expandable bellows (3, 63).

10. The expandable medical implant (1, 31, 41, 51, 61, 71, 81, 91) of claim 1 wherein the laterally rigid and longitudinally expandable component is a telescoping body (23, 33, 73, 83, 39).

11. The expandable medical implant (1, 31, 41, 51, 61, 71, 81, 91) of claim 1 wherein the laterally rigid and longitudinally expandable component (3, 23, 33, 43, 53, 63, 73, 83, 93) is integral with a portion of the laterally and longitudinally expandable membrane (5, 25, 35, 45, 55, 65, 75, 85, 95) to restrict lateral expansion of the laterally and longitudinally expandable membrane (5, 25, 35, 45, 55, 65, 75, 85, 95) in the direction of the laterally rigid and longitudinally expandable component (3, 23, 33, 43, 53, 63, 73, 83, 93) that is integrated into the laterally and longitudinally expandable membrane (5, 25, 35, 45, 55, 65, 75, 85, 95).

12. The expandable medical implant (1, 31, 41, 51, 61, 71, 81, 91) of claim 1 wherein the laterally rigid and longitudinally expandable component (3, 23, 33, 43, 53, 63, 73, 83, 93) receives a force to drive expansion of the expandable medical implant (1, 31, 41, 51, 61, 71, 81, 91).

13. The expandable medical implant (1, 31, 41, 51, 61, 71, 81, 91) of claim 1, further comprising a fill material (100) for filling at least the laterally and longitudinally expandable membrane (5, 25, 35, 45, 55, 65, 75, 85, 95).

## Patentansprüche

1. Ein expandierbares medizinisches Implantat (1, 31, 41, 51, 61, 71, 81, 91) zum Abstützen von Skelettstrukturen, aufweisend:
eine seitlich und längs expandierbare Membran (5, 25, 35, 45, 55, 65, 75, 85, 95), die ein Volumen definiert und eine obere Fläche, eine entgegengesetzte untere Fläche und eine erste Seite zwischen der oberen und der unteren Fläche aufweist,
und eine seitlich steife und längs expandierbare Komponente (3, 23, 33, 43, 53, 63, 73, 83, 93), die an mindestens einem Punkt mit der ersten Seite verbunden ist, wobei die seitlich steife und längs expandierbare Komponente (3, 23, 33, 43, 53, 63, 73, 83, 93) im Wesentlichen innerhalb des Volumens der seitlich und längs expandierbaren Membran (5, 25, 35, 45, 55, 65, 75, 85, 95) enthalten ist,
wobei die seitlich und längs expandierbare Membran (5, 25, 35, 45, 55, 65, 75, 85, 95) an ihrer ersten Seite mit der seitlich steifen und längs expandierbaren Komponente (3, 23, 33, 43, 53, 63, 73, 83, 93) verbunden ist, um ein seitliches Expandieren der seitlich und längs expandierbaren Membran (5, 25, 35, 45, 55, 65, 75, 85, 95) in zumindest einer ersten Richtung zu beschränken.

2. Das expandierbare medizinische Implantat (1, 31, 41, 51, 61, 71, 81, 91) gemäß Anspruch 1, wobei die seitlich und längs expandierbare Membran (5, 25, 35, 45, 55, 65, 75, 85, 95) zumindest teilweise eine permeable seitlich und längs expandierbare Membran (5, 25, 35, 45, 55, 65, 75, 85, 95) ist.

3. Das expandierbare medizinische Implantat (1, 31, 41, 51, 61, 71, 81, 91) gemäß Anspruch 1, wobei die seitlich steife und längs expandierbare Komponente (3, 23, 33, 43, 53, 63, 73, 83, 93) mit der seitlich und längs expandierbaren Membran (5, 25, 35, 45, 55, 65, 75, 85, 95) an mehr als einem Punkt entlang der ersten Seite der seitlich und längs expandierbaren Membran (5, 25, 35, 45, 55, 65, 75, 85, 95) verbunden ist.

4. Das expandierbare medizinische Implantat (1, 31, 41, 51, 61, 71, 81, 91) gemäß Anspruch 1, wobei die seitlich steife und längs expandierbare Komponente (3, 23, 33, 43, 53, 63, 73, 83, 93) mit der seitlich und längs expandierbaren Membran (5, 25, 35, 45, 55, 65, 75, 85, 95) an einem ersten Punkt nahe bei der oberen Fläche der seitlich und längs expandierbaren Membran (5, 25, 35, 45, 55, 65, 75, 85, 95) und an einem zweiten Punkt nahe bei der unteren Fläche der seitlich und längs expandierbaren Membran (5, 25, 35, 45, 55, 65, 75, 85, 95) verbunden ist.

5. Das expandierbare medizinische Implantat (1, 31, 41, 51, 61, 71, 81, 91) gemäß Anspruch 1, wobei die seitlich steife und längs expandierbare Komponente (3, 23, 33, 43, 53, 63, 73, 83, 93) mit der seitlich und längs expandierbaren Membran (5, 25, 35, 45, 55, 65, 75, 85, 95) durchgehend entlang der ersten Seite der seitlich und längs expandierbaren Membran (5, 25, 35, 45, 55, 65, 75, 85, 95) verbunden ist.

6. Das expandierbare medizinische Implantat (1, 31, 41, 51, 61, 71, 81, 91) gemäß Anspruch 1, wobei die seitlich steife und längs expandierbare Komponente (3, 23, 33, 43, 53, 63, 73, 83, 93) in mindestens eine erste Seite der seitlich und längs expandierbaren Membran (5, 25, 35, 45, 55, 65, 75, 85, 95) eingebettet ist.

7. Das expandierbare medizinische Implantat (1, 31, 41, 51, 61, 71, 81, 91) gemäß Anspruch 1, ferner aufweisend einen Klebstoff zwischen der seitlich und längs expandierbaren Membran (5, 25, 35, 45, 55, 65, 75, 85, 95) und der seitlich steifen und längs expandierbaren Komponente (3, 23, 33, 43, 53, 63, 73, 83, 93).

8. Das expandierbare medizinische Implantat (1, 31, 41, 51, 61, 71, 81, 91) gemäß Anspruch 1, ferner aufweisend mindestens einen Gurt zwischen der seitlich und längs expandierbaren Membran (5, 25, 35, 45, 55, 65, 75, 85, 95) und der seitlich steifen und längs expandierbaren Komponente (3, 23, 33, 43, 53, 63, 73, 83, 93).

9. Das expandierbare medizinische Implantat (1, 31, 41, 51, 61, 71, 81, 91) gemäß Anspruch 1, wobei die seitlich steife und längs expandierbare Komponente (3, 23, 33, 43, 53, 63, 73, 83, 93) ein linear expandierbarer Balg (3, 63) ist.

10. Das expandierbare medizinische Implantat (1, 31, 41, 51, 61, 71, 81, 91) gemäß Anspruch 1, wobei die seitlich steife und längs expandierbare Komponente ein Teleskopkörper (23, 33, 73, 83, 39) ist.

11. Das expandierbare medizinische Implantat (1, 31, 41, 51, 61, 71, 81, 91) gemäß Anspruch 1, wobei die seitlich steife und längs expandierbare Komponente (3, 23, 33, 43, 53, 63, 73, 83, 93) mit einem Abschnitt der seitlich und längs expandierbaren Membran (5, 25, 35, 45, 55, 65, 75, 85, 95) integral ist, um das seitliche Expandieren der seitlich und längs expandierbaren Membran (5, 25, 35, 45, 55, 65, 75, 85, 95) in der Richtung der seitlich steifen und längs expandierbaren Komponente (3, 23, 33, 43, 53, 63, 73, 83, 93), die in die seitlich und längs expandierbare Membran (5, 25, 35, 45, 55, 65, 75, 85, 95) integriert ist, zu begrenzen.

12. Das expandierbare medizinische Implantat (1, 31, 41, 51, 61, 71, 81, 91) gemäß Anspruch 1, wobei die seitlich steife und längs expandierbare Komponente (3, 23, 33, 43, 53, 63, 73, 83, 93) eine Kraft aufnimmt, um das Expandieren des expandierbaren medizinischen Implantats (1, 31, 41, 51, 61, 71, 81, 91) anzutreiben.

13. Das expandierbare medizinische Implantat (1, 31, 41, 51, 61, 71, 81, 91) gemäß Anspruch 1, ferner aufweisend ein Füllmaterial (100) zum Füllen von zumindest der seitlich und längs expandierbaren Membran (5, 25, 35, 45, 55, 65, 75, 85, 95).

## Revendications

1. Un implant médical expansible (1, 31, 41, 51, 61, 71, 81, 91) pour supporter des structures de squelette, comportant :
une membrane expansible latéralement et longitudinalement (5, 25, 35, 45, 55, 65, 75, 85, 95) définissant un volume et ayant une surface supérieure, une surface inférieure opposée et une première face entre les surfaces supérieure et inférieure,
et un composant rigide latéralement et expansible longitudinalement (3, 23, 33, 43, 53, 63, 73, 83, 93), relié au moins à un point avec la première face, le composant rigide latéralement et expansible longitudinalement (3, 23, 33, 43, 53, 63, 73, 83, 93) étant essentiellement contenu à l'intérieur du volume de la membrane expansible latéralement et longitudinalement (5, 25, 35, 45, 55, 65, 75, 85, 95),
dans lequel la membrane expansible latéralement et longitudinalement (5, 25, 35, 45, 55, 65, 75, 85, 95) est, à sa première face, reliée avec le composant rigide latéralement et expansible longitudinalement (3, 23, 33, 43, 53, 63, 73, 83, 93), afin de délimiter l'expansion latérale de la membrane expansible latéralement et longitudinalement (5, 25, 35, 45, 55, 65, 75, 85, 95) dans au moins une première direction.

2. L'implant médical expansible (1, 31, 41, 51, 61, 71, 81, 91) selon la revendication 1, dans lequel la membrane expansible latéralement et longitudinalement (5, 25, 35, 45, 55, 65, 75, 85, 95) est, au moins en partie, une membrane perméable expansible latéralement et longitudinalement (5, 25, 35, 45, 55, 65, 75, 85, 95).

3. L'implant médical expansible (1, 31, 41, 51, 61, 71, 81, 91) selon la revendication 1, dans lequel le composant rigide latéralement et expansible longitudinalement (3, 23, 33, 43, 53, 63, 73, 83, 93) est relié avec la membrane expansible latéralement et longitudinalement (5, 25, 35, 45, 55, 65, 75, 85, 95) à plus d'un point le long de la première face de la membrane expansible latéralement et longitudinalement (5, 25, 35, 45, 55, 65, 75, 85, 95).

4. L'implant médical expansible (1, 31, 41, 51, 61, 71, 81, 91) selon la revendication 1, dans lequel le composant rigide latéralement et expansible longitudinalement (3, 23, 33, 43, 53, 63, 73, 83, 93) est relié avec la membrane expansible latéralement et longitudinalement (5, 25, 35, 45, 55, 65, 75, 85, 95) à un premier point près de la surface supérieure de la membrane expansible latéralement et longitudinalement (5, 25, 35, 45, 55, 65, 75, 85, 95) et à un deuxième point près de la surface inférieure de membrane expansible latéralement et longitudinalement (5, 25, 35, 45, 55, 65, 75, 85, 95).

5. L'implant médical expansible (1, 31, 41, 51, 61, 71, 81, 91) selon la revendication 1, dans lequel le composant rigide latéralement et expansible longitudinalement (3, 23, 33, 43, 53, 63, 73, 83, 93) est relié avec la membrane expansible latéralement et longitudinalement (5, 25, 35, 45, 55, 65, 75, 85, 95) de façon continue le long de la première face de la membrane expansible latéralement et longitudinalement (5, 25, 35, 45, 55, 65, 75, 85, 95).

6. L'implant médical expansible (1, 31, 41, 51, 61, 71, 81, 91) selon la revendication 1, dans lequel le composant rigide latéralement et expansible longitudinalement (3, 23, 33, 43, 53, 63, 73, 83, 93) est encastré dans au moins une première face de la membrane expansible latéralement et longitudinalement (5, 25, 35, 45, 55, 65, 75, 85, 95).

7. L'implant médical expansible (1, 31, 41, 51, 61, 71, 81, 91) selon la revendication 1, comportant en outre un adhésif entre la membrane expansible latéralement et longitudinalement (5, 25, 35, 45, 55, 65, 75, 85, 95) et le composant rigide latéralement et expansible longitudinalement (3, 23, 33, 43, 53, 63, 73, 83, 93).

8. L'implant médical expansible (1, 31, 41, 51, 61, 71, 81, 91) selon la revendication 1, comportant en outre au moins une ceinture entre la membrane expansible latéralement et longitudinalement (5, 25, 35, 45, 55, 65, 75, 85, 95) et le composant rigide latéralement et expansible longitudinalement (3, 23, 33, 43, 53, 63, 73, 83, 93).

9. L'implant médical expansible (1, 31, 41, 51, 61, 71, 81, 91) selon la revendication 1, dans lequel le composant rigide latéralement et expansible longitudinalement (3, 23, 33, 43, 53, 63, 73, 83, 93) est un soufflet expansible linéairement.

10. L'implant médical expansible (1, 31, 41, 51, 61, 71, 81, 91) selon la revendication 1, dans lequel le composant rigide latéralement et expansible longitudinalement (3, 23, 33, 43, 53, 63, 73, 83, 93) est un corps télescopique.

11. L'implant médical expansible (1, 31, 41, 51, 61, 71, 81, 91) selon la revendication 1, dans lequel lé composant rigide latéralement et expansible longitudinalement (3, 23, 33, 43, 53, 63, 73, 83, 93) est solidaire d'une partie de la membrane expansible latéralement et longitudinalement (5, 25, 35, 45, 55, 65, 75, 85, 95), afin de délimiter l'expansion latérale de la membrane expansible latéralement et longitudinalement (5, 25, 35, 45, 55, 65, 75, 85, 95) dans le sens du composant rigide latéralement et expansible longitudinalement (3, 23, 33, 43, 53, 63, 73, 83, 93) qui est intégré dans la membrane expansible latéralement et longitudinalement (5, 25, 35, 45, 55, 65, 75, 85, 95).

12. L'implant médical expansible (1, 31, 41, 51, 61, 71, 81, 91) selon la revendication 1, dans lequel le composant rigide latéralement et expansible longitudinalement (3, 23, 33, 43, 53, 63, 73, 83, 93) reçoit une force, afin d'entraîner l'expansion de l'implant médical expansible (1, 31, 41, 51, 61, 71, 81, 91).

13. L'implant médical expansible (1, 31, 41, 51, 61, 71, 81, 91) selon la revendication 1, comportant en outre un matériau de remplissage (100) pour remplir au moins la membrane expansible latéralement et longitudinalement (5, 25, 35, 45, 55, 65, 75, 85, 95).
